# EUROPÄISCHE PATENTSCHRIFT

(11) **EP 3 685 770 B1**
(45) Veröffentlichungstag und Bekanntmachung des Hinweises auf die Patenterteilung: **11.09.2024**
(21) Anmeldenummer: 19209798.8
(22) Anmeldetag: 18.11.2019
(51) Int. Cl.: A61B 17/14

(54) **CHIRURGISCHES WERKZEUG MIT EFFEKTORAUFNAHME**
SURGICAL TOOL WITH EFFECTOR ACCOMODATION
OUTIL CHIRURGICAL POURVU DE LOGEMENT D'EFFECTEUR

(30) Priorität: 29.11.2018 DE 102018130381
(43) Veröffentlichungstag der Anmeldung: 29.07.2020
(73) Patentinhaber: Aesculap AG, 78532 Tuttlingen (DE)
(72) Erfinder: HÖGERLE, Roland-Alois, 78532 Tuttlingen (DE); KRÄMER, Frank, 78532 Tuttlingen (DE); SCHAZ, Uwe, 78579 Neuhausen (DE)
(74) Vertreter: Winter, Brandl - Partnerschaft mbB

(56) Entgegenhaltungen:
- EP-A1- 0 962 192
- EP-A1- 2 499 978
- EP-A1- 3 050 521
- EP-A2- 1 974 881
- DE-A1- 102012 014 008
- US-A- 2 741 248
- US-A1- 2001 021 854
- US-A1- 2006 123 959
- US-A1- 2010 168 751
- US-A1- 2017 065 286

## Beschreibung

### Technisches Gebiet

Die vorliegende Offenbarung betrifft ein chirurgisches Werkzeug mit einer Effektoraufnahme am Werkzeugschaft.

### Hintergrund der Erfindung

Es ist ein chirurgisches Werkzeug, insbesondere chirurgische Transversalsäge, Stichsäge oder chirurgisches Ultraschallschneidwerkzeug, bekannt, das einen, vorzugsweise schlanken, Werkzeugschaft aufweist, der an seinem proximalen Ende eine Kopplungsstruktur zur Kopplung mit einem Handstück aufweist und der zur reversiblen Kopplung des Schafts mit einem Effektor an seinem distalen Ende eine Effektoraufnahme aufweist. Die Effektoraufnahme nimmt den Effektor üblicherweise so auf, dass Effektor und Werkzeugschaft einen vorbestimmten Winkel einschließen, der unveränderlich ist. Werkzeugschäfte in unterschiedlichen Längen sind mit dem Handstück koppelbar. Je nach Indikation werden Effektoren benötigt, die unterschiedliche Winkel mit dem Werkzeugschaft einschließen. Daher muss der Anwender verschiedene Werkzeugschaft-/Effektoreinheiten vorhalten.

### Stand der Technik

Aus dem Stand der Technik sind Werkzeuge bekannt, deren Werkzeugschaft und Effektor unlösbar miteinander verbunden sind, was zu hohen Herstellungskosten führt, da an jedem Werkzeugschaft die Kopplungsgeometrie zum Koppeln mit dem Handstück angebracht werden muss. Ist der Effektor beschädigt oder defekt, muss die Werkzeugschaft-/Effektoreinheit ausgetauscht werden.

Es gibt daher auch Werkzeuge, die einen wiederverwendbaren Werkzeugschaft mit austauschbarem Effektor vorsehen. Außerdem ist beispielsweise aus der Druckschrift EP 1 765 188 B1 ein chirurgisches Werkzeug bekannt, dessen Effektor (Sägeblatt) direkt und reversibel/austauschbar mit dem Handstück gekoppelt ist. Ein ähnlicher Werkzeugaufbau ist auch aus US 5 439 472 A bekannt.

Außerdem ist aus EP 0 962 192 A1 ein Instrument mit einem Handstück, welches einen Handstückschaft und eine Haltevorrichtung aufweist, bekannt. Diese Haltevorrichtung kann ein Werkzeug aufnehmen, welches einen Werkzeugschaft und einen Effektor aufweist. Dabei weist die Haltevorrichtung ein Steckloch zur Aufnahme einer Fixiervorrichtung auf, die sich am Werkzeugschaft befindet,. Zwischen Werkzeugschaft und Effektor sind unterschiedliche Winkelstellungen möglich, indem mehrere, unterschiedlich angewinkelte Effektoren mit dem Werkzeugschaft verbunden werden können.

Diese Werkzeuge mit auswechselbarem Effektor, der mit dem Werkzeugscahft gekoppelt ist, haben den Nachteil, dass ein Anwender mehrere Werkzeugschaft-/Effektoreinheiten für verschiedene Winkelstellungen vorhalten muss. Bei einem Wechsel auf einen anderen Effektor ist daher normalerweise auch ein Werkzeugschaftwechsel erforderlich, der oftmals aufwendig unter Verwendung eines Schlüssels oder dergleichen erfolgt.

Es ist daher ein Ziel der Erfindung, das obige Problem zu lösen und ein chirurgisches Werkzeug vorzusehen, das die Verwendung einer Werkzeugschaft-/Effektoreinheit für mindestens zwei definierte Winkelstellungen zwischen Werkzeugschaft und Effektor ermöglicht.

### Zusammenfassung der Erfindung

Die Erfindung sieht daher ein chirurgisches Werkzeug vor, insbesondere chirurgische Transversalsäge, Stichsäge oder chirurgisches Ultraschallschneidwerkzeug, mit einem Effektor und einem, insbesondere im Vergleich mit einem Handstück schlanken, Werkzeugschaft, der an seinem proximalen Ende eine Kopplungsstruktur zur Kopplung mit dem Handstück aufweist und der zur reversiblen Kopplung des Schafts mit dem Effektor an seinem distalen Ende eine Effektoraufnahme aufweist, welche zur Längsachse des Schafts in einem vorbestimmten ersten, insbesondere spitzen, Winkel angeordnet ist. Dabei kann der Effektor über einen Kopplungsabschnitt derart mit der Effektoraufnahme gekoppelt werden, dass der Effektor und der Werkzeugschaft mit der Effektoraufnahme in zwei unterschiedliche Winkelstellungen, nämlich in genau zwei im 180° Drehabstand vorgesehene Drehpositionen, zueinander bringbar sind.

In anderen Worten betrifft die Erfindung ein chirurgisches Werkzeug, insbesondere chirurgische Transversalsäge, Stichsäge oder chirurgisches Ultraschallschneidwerkzeug, mit einem Effektor und einem, insbesondere im Vergleich mit einem Handstück schlanken, Werkzeugschaft, der an seinem proximalen Ende eine Kopplungsstruktur zur Kopplung mit dem Handstück aufweist und der zur reversiblen Kopplung des Schafts mit dem Effektor/Werkzeugkopf an seinem distalen Ende eine Effektoraufnahme/Werkzeugkupplung/Schublade/Werkzeugaufnahme aufweist, welche zur Längsachse des Schafts in einem definierten ersten Winkel größer 0° (gekröpft) angeordnet ist. Der Effektor weist einen Kopplungsabschnitt zur reversiblen Kopplung mit dem Werkzeugschaft/dessen Effektoraufnahme sowie ein Arbeitsende auf. Durch eine Ausgestaltung der Effektoraufnahme derart, dass der Effektor in genau zwei im 180° Drehabstand vorgesehenen Drehpositionen relativ zum Werkzeugschaft mit der Effektoraufnahme koppelbar ist oder durch eine Variation des ersten Winkels/Kröpfung, bspw. durch Verbiegen der Effektoraufnahme relativ zum Werkzeugschaft, lassen sich nach der Kopplung mit dem Effektor zwei definierte Winkelstellungen zwischen Effektor(-ende) und dem Werkzeugschaft erzeugen.

Der Vorteil der Erfindung liegt somit darin, dass durch einfaches Ändern des Drehabstandes zwischen Effektor und Werkzeugschaft/dessen Effektoraufnahme zumindest zwei definierte Winkelstellungen einstellbar sind. Damit ist der Werkzeugschaft wiederverwendbar und das Werkzeug ohne großen Aufwand für verschiedene Anforderungen verwendbar.

Eine alternative Ausführungsform sieht vor, dass der Effektor neben dem Kopplungsabschnitt ein Arbeitsende aufweist, welches mit dem Kopplungsabschnitt einen vorbestimmten zweiten Winkel einschließt.

Damit ist das Arbeitsende zu dem Kopplungsabschnitt unter einem definierten zweiten Winkel gebogen/gewinkelt. Durch Variation des ersten Winkels oder des zweiten Winkels, bspw. durch Verbiegen, lassen sich beliebige weitere sinnvolle Winkelkombinationen (mehr als zwei) erzeugen. Der Effektor, der für das erfindungsgemäße Werkzeug verwendet wird, ist aufgrund seiner einfachen Ausführung günstig herstellbar und kann als Einmaleffektor oder aber auch so lange verwendet werden, bis der Effektor abgenutzt ist.

Weiterhin ist denkbar, dass der Kopplungsabschnitt des Effektors mit der Effektoraufnahme eine Hinterschneidung ausbildet.

Durch eine derartige Hinterschneidung sitzt der Effektor fest in der Effektoraufnahme und kann sich auch bei Kraftausübung nicht einfach in einer unerwünschten Art und Weise verschieben.

Außerdem kann vorgesehen sein, dass die Effektoraufnahme eine erste Kopplungsvorrichtung aufweist, die komplementär zu einer zweiten Kopplungsvorrichtung am Kopplungsabschnitt des Effektors ausgebildet ist, sodass die erste und die zweite Kopplungsvorrichtung als Rastverbindung oder Schnappverbindung oder Steckverbindung zusammenwirken.

Durch eine derartige Verbindung zwischen Effektoraufnahme und Effektor kann eine sichere Aufnahme des Effektors in der Effektoraufnahme garantiert werden und ein unerwünschtes Lösen des Effektors aus der Effektoraufnahme wird unterdrückt.

Weiter erfindungsgemäß ist ein chirurgisches Werkzeugsystem vorgesehen, das ein chirurgisches Werkzeug, wie es oben beschrieben ist, und ein Handstück zur Bedienung des chirurgischen Werkzeugs aufweist, wobei der Effektor über einen Kopplungsabschnitt reversibel mit der Effektoraufnahme koppelbar ist und der Effektor und der Werkzeugschaft in zumindest zwei unterschiedliche Winkelstellungen zueinander bringbar sind.

### Kurzbeschreibung der Figuren

Im Folgenden sind Ausführungsformen des erfindungsgemäßen chirurgischen Werkzeugs unter Bezug auf die beigefügten Zeichnungen im Detail beschrieben. Dabei werden gleichen Elementen dieselben Bezugszeichen zugewiesen. Die Ausführungsformen sind nur beispielhaft und die Erfindung ist nicht darauf begrenzt.
Fig. 1 ist eine perspektivische Ansicht eines chirurgischen Werkzeugs mit Werkzeugschaft und Effektor in einer ersten Ausführungsform;
Fig. 2A ist eine Draufsicht auf einen Ausschnitt des chirurgischen Werkzeugs, die den Effektor und eine Effektoraufnahme der ersten Ausführungsform im Nicht-Koppelzustand zeigt;
Fig. 2B ist eine Draufsicht, die den Effektor und die Effektoraufnahme aus Fig. 2A im Koppelzustand zeigt;
Fig. 2C ist eine Draufsicht, die den Effektor und die Effektoraufnahme, deren Drehposition relativ zueinander im Vergleich zu Fig. 2A um 180° geändert ist, im Nicht-Koppelzustand zeigt;
Fig. 2D ist eine Draufsicht, die den Effektor und die Effektoraufnahme aus Fig. 2C im Koppelzustand zeigt;
Fig. 3 ist zeigt eine Längsschnittansicht des Effektors und der Effektoraufnahme im Kopplungszustand;
Fig. 4A ist eine Draufsicht auf das chirurgische Werkzeug in einer alternativen Ausführungsform, die den Effektor und die Effektoraufnahme im Nicht-Koppelzustand zeigt;
Fig. 4B ist eine Draufsicht, die den Effektor und die Effektoraufnahme aus Fig. 4A im Koppelzustand zeigt;
Fig. 4C ist eine Draufsicht, die den Effektor und die Effektoraufnahme, deren Drehposition relativ zueinander im Vergleich zu Fig. 4B um 180° geändert ist, im Koppelzustand zeigt;

### Beschreibung der Ausführungsformen

Nachstehend werden Ausführungsformen der vorliegenden Offenbarung auf der Basis der zugehörigen Figuren beschrieben.

### Erste Ausführungsform

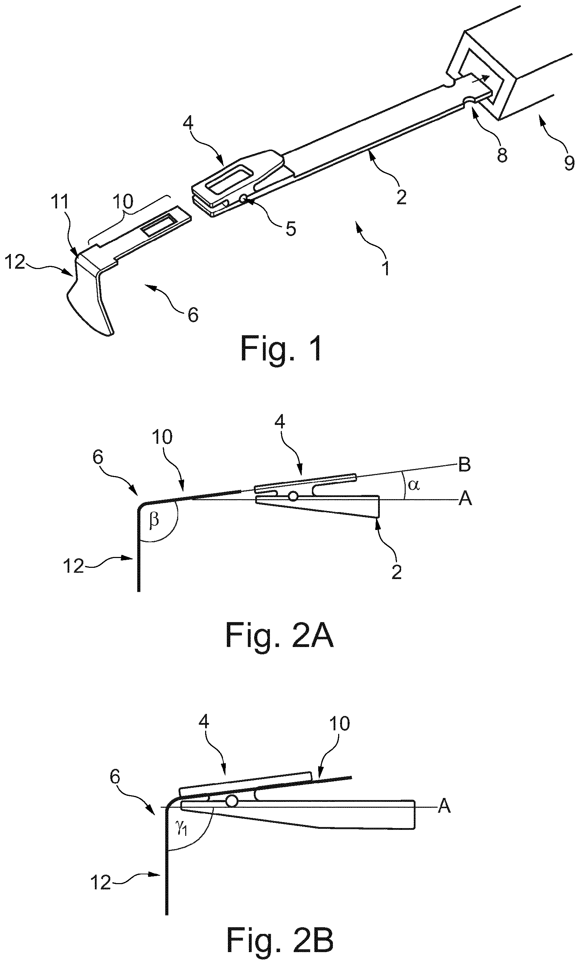

Fig. 1 zeigt eine perspektivische Ansicht eines chirurgischen Werkzeugs 1, das hier als Transversalsäge ausgeführt ist. Das Werkzeug 1 weist einen Werkzeugschaft 2 auf, der hier flach ausgeführt ist und in seiner Grundform rechteckig ist. Es ist aber ebenfalls möglich, dass der Werkzeugschaft 2 anderweitig, bspw. rund, ausgeführt ist. Der Werkzeugschaft 2 weist an seinem distalen Ende eine Effektoraufnahme bzw. Sägeblattaufnahme 4 auf, die in dieser Ausführungsform einstückig und starr mit dem Werkzeugschaft 2 verbunden ist. Die Effektoraufnahme 4 ist dazu vorgesehen, einen Effektor 6 aufzunehmen. Der Werkzeugschaft 2 weist an seinem proximalen Ende eine Kopplungsstruktur 8 zur Kopplung mit einem Handstück 9 auf. Die Kopplungsstuktur 8 ist hier als zur Längsachse A des Werkzeugschaftes 2 hin vertiefte Einkerbung ausgeführt, die nahe dem proximalen Ende des Werkzeugschaftes 2 angeordnet ist. Die Effektoraufnahme 4 hat eine rechteckige Grundform, die an dem Ende, das zum proximalen Ende des Werkzeugschaftes hin gerichtet ist, leicht konisch zuläuft. Der Effektor 6 ist hier als Säge ausgeführt und über den Kopplungsabschnitt 10 mit der Effektoraufnahme 4 koppelbar. Zum Kopplungsabschnitt 10 (fast senkrecht) über ein Scharnier 11 abgewinkelt ist das Arbeitsende 12 des Effektors 6 angeordnet, welches hier als Sägeblatt 12 ausgeführt ist. Der Kopplungsabschnitt 10 hat eine rechteckige Grundform.

Fig. 2A zeigt den Effektor 6 und die Effektoraufnahme 4, die nicht miteinander im Eingriff stehen, d.h. in einem Nicht-Koppelzustand sind. Eine Längsachse B der Effektoraufnahme 4 schließt mit einer Längsachse A des Werkzeugschaftes 2 einen ersten Winkel α ein, der hier ein spitzer Winkel ist und vorzugsweise einen Wert von 7,5° hat. Der Kopplungsabschnitt 10 bildet mit dem Sägeblatt 12 einen zweiten Winkel β aus, der in dieser Darstellung größer als 90° ist, vorzugsweise 97,5°, aber auch eine andere Winkelgröße haben kann. Es ist zu erkennen, dass der Bereich des Werkzeugschaftes 2 und die Effektoraufnahme 4 in der Draufsicht eine konische Außenform aufweisen, die zum distalen Ende hin zuläuft.

Fig. 2B zeigt den Effektor 6 und die Effektoraufnahme 4, die ausgehend von Fig. 2A miteinander in Eingriff stehen, d.h. in einem Koppelzustand sind. Im Koppelzustand bilden der Koppelabschnitt 10 des Effektors 6 und die Effektoraufnahme 4 eine Hinterschneidung aus. Ein Winkel γ₁, den die Längsachse A des Werkzeugschaftes 2 und das Sägeblatt 12 einschließen, ergibt sich hier aus der Subtraktion des ersten Winkels α vom zweiten Winkel β. In dem Fall, in dem der erste Winkel α einen Wert von 7,5° hat und der zweite Winkel β einen Wert von 97,5° hat, ergibt sich für den Winkel γ₁ ein Wert von 90°.

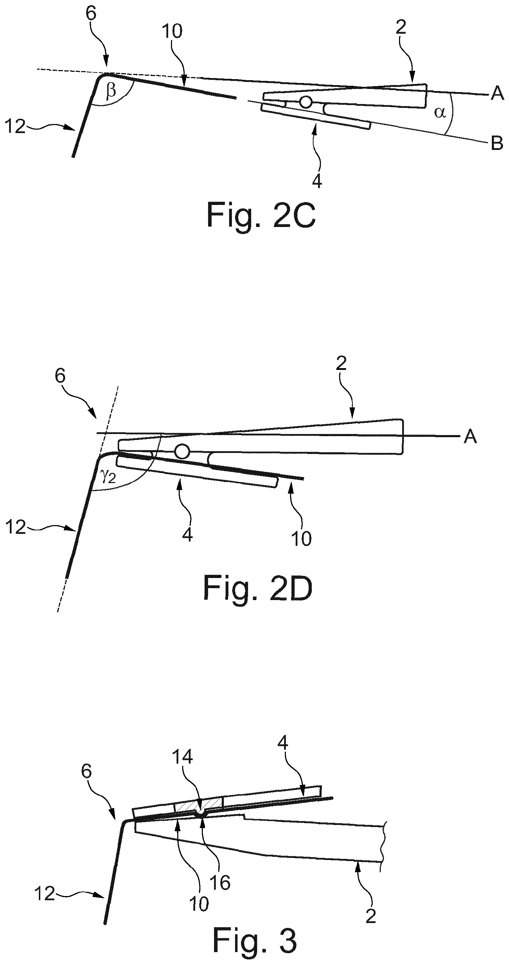

Fig. 2C zeigt den Effektor 6 und die Effektoraufnahme 4, die in dem Nicht-Koppelzustand sind. Ausgehend von Fig. 2A ist die Effektoraufnahme 4 und damit der Werkzeugschaft 2 um dessen Längsachse A um 180° gedreht worden. Damit ist der Drehabstand zwischen Effektor und Effektoraufnahme im Vergleich zur relativen Positionierung in Fig. 2A relativ zueinander um 180° geändert. Die Ausrichtung des Effektors 6 orientiert sich entlang der Verlaufsichtung der Effektoraufnahme.

Fig. 2D zeigt den Effektor 6 und die Effektoraufnahme 4, die im Koppelzustand miteinander sind. Der Effektor 6 ist so in die Effektoraufnahme 4 eingeschoben, dass ein Winkel γ₂, den die Längsachse A und das Sägeblatt 12 einschließen, sich aus der Addition des ersten Winkels α und des zweiten Winkels β ergibt. In dem Fall, in dem der erste Winkel α einen Wert von 7,5° hat und der zweite Winkel β einen Wert von 97,5° hat, ergibt sich für den Winkel γ₂ ein Wert von 105°.

Aus dem Vergleich von Fig. 2B und Fig. 2D wird klar, dass es durch eine einfache Drehung des Werkzeugschaftes 2 (um dessen Längsachse A, C relativ zum Effektor 6) und die anschließende Kopplung von Effektor 6 und Effektoraufnahme 4 und damit durch Änderung des Drehabstandes zwischen Effektor 6 und Effektoraufnahme 4 möglich ist, zwei Winkelstellungen, d.h. den ersten Winkel α und den zweiten Winkel β, der sich vom ersten Winkel α unterscheidet, zwischen der Längsachse A des Werkzeugschaftes 2 und dem Effektor 6 bzw. dem Arbeitsende 12 zu erzeugen. Ein Wechsel zwischen den zwei Winkelstellungen kann somit sehr einfach und schnell durchgeführt werden. Je nach Einschub des Sägeblattes/Arbeitsendes 12 lassen sich in dieser Ausführungsform zwei definierte Winkelstellungen erzeugen.

Durch einfaches Verbiegen der Effektoraufnahme 4 gegenüber dem Werkzeugschaft 2 und/oder durch einfaches Verbiegen des Arbeitsendes 12 zum Kopplungsabschnitt 10 des Effektors 6 oder aber mithilfe eines entsprechenden Scharniers 11 am Effektor können die ersten und zweiten Winkel jeweils verändert werden, sodass sich beliebige weitere Winkelkombinationen und damit Winkelstellungen erzeugen lassen.

Fig. 3 zeigt Fig. 2B in einer Längsschnittansicht. Der Kopplungsabschnitt 10 des Effektors 6 ist in die Effektoraufnahme 4 aufgenommen und damit im Kopplungszustand. Es ist zu erkennen, dass die Effektoraufnahme 4 eine erste Kopplungsvorrichtung 14 aufweist. Diese erste Kopplungsvorrichtung 14 ist an der Innenseite der Effektoraufnahme 4 vorgesehen und in Form einer Erhebung/Schiene ausgebildet, die sich vorzugsweise über die gesamte Breite der Effektoraufnahme 4 erstreckt. Mit der ersten Kopplungsvorrichtung 14 der Effektoraufnahme 4 steht eine zweite Kopplungsvorrichtung 16 des Effektors 6 im Eingriff, d.h., dass die erste und die zweite Kopplungsvorrichtung 14, 16 in dieser Ausführung in einem Schnappeingriff miteinander stehen. Die Kopplungsvorrichtung 16 ist oberseitig am proximalen Ende des Kopplungsabschnittes 10 des Effektors 6 in Form einer Nut/Rille ausgebildet, die sich vorzugweise über die gesamte Breite des Kopplungsabschnittes 10 erstreckt. Durch das Eingreifen der ersten Kopplungsvorrichtung 14 in die zweite Kopplungsvorrichtung 16 wird ein unerwünschtes Lösen des Effektors 6 aus der Effektoraufnahme 4 unterdrückt. Es ist zum Koppeln/zum Entkoppeln zwischen Effektoraufnahme 4 und Effektor 6 ein erhöhter Kraftaufwand in die Verlaufsrichtung der Effektoraufnahme 4 nötig, um den Schnappeingriff zwischen der ersten Kopplungsvorrichtung 14 und der zweiten Kopplungsvorrichtung 16 herzustellen/zu lösen. Auch wenn es nicht dargestellt ist, ist es denkbar, dass die Kopplungsvorrichtungen 14, 16 in einen Rasteingriff eintreten und dementsprechend anders als dargestellt ausgebildet sind.

### Zweite Ausführungsform

Nachstehend wird eine zweite Ausführungsform des chirurgischen Werkzeugs beschrieben, die in ihrem Wirkprinzip ähnlich der ersten Ausführungsform ist. Es werden hierbei hauptsächlich die Unterschiede zur ersten Ausführungsform dargestellt.

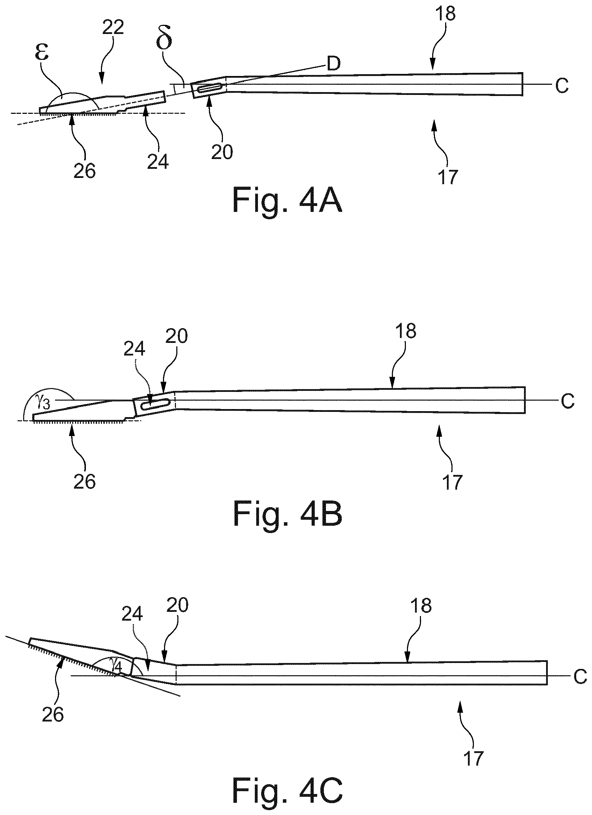

Fig. 4A zeigt ein chirurgisches Werkzeug in einer zweiten Ausführungsform, welches hier ein Stichsägewerkzeug ist. Der Werkzeugschaft 18 hat eine rechteckige Grundform und läuft zu seinem distalen Ende hin leicht konisch zu. Am distalen Ende weist der Werkzeugschaft 18 die Effektoraunahme 20 auf, deren Längsachse D in einem ersten Winkel δ zu einer Längsachse C des Werkzeugschaftes 18 abgewinkelt ist. Weiterhin ist der Effektor 22 dargestellt, dessen Kopplungsabschnitt 24 zum Arbeitsende 26 in einem zweiten Winkel ε gewinkelt ist. Das Arbeitsende 26 hat eine dreieckige Grundform und an der Unterseite eine Reihe einer Vielzahl an Sägezähnen. Auch wenn es nicht dargestellt ist, kann der Werkzeugschaft 2 an seinem proximalen Ende eine Kopplungsstruktur zu Kopplung mit einem Handstück 9 aufweisen.

Fig. 4B zeigt den Effektor 22 im Kopplungszustand mit der Effektoraufnahme 20. Der Kopplungsabschnitt 24 ist in die Effektoraufnahme 20 eingeschoben, welche hohl ist. Auch wenn es nicht dargestellt ist, kann die Effektoraufnahme 20 eine erste Kopplungsvorrichtung und der Kopplungsabschnitt 24 des Effektors 22 eine zweite Kopplungsvorrichtung ähnlich den ersten und zweiten Kopplungsvorrichtungen 14, 16 der ersten Ausführungsform aufweisen. Ein Winkel γ₃, den die Sägezahnreihe des Arbeitsendes 26 und die Längsachse C des Werkzeugschaftes 18 einschließen, ergibt sich aus der Addition des ersten Winkels δ und des zweiten Winkels ε.

Fig. 4C zeigt die ausgehend von Fig. 4B um 180° gedrehte Stichsäge 17 und zweigt damit den ausgehend von Fig. 4B um 180° geänderten Drehabstand zwischen Effektor 22 und Effektoraufnahme 20. Der Effektor 22 wird in dieser Ausführungsform vor der Drehung aus dem Kopplungszustand gelöst und nach der Drehung wieder in den Kopplungszustand gebracht, damit das Arbeitsende stets nach unten zeigt. Ein Winkel γ₄, den die Sägezahnreihe des Arbeitsendes 26 und die Längsachse C des Werkzeugschaftes 18 einschließen, ergibt sich aus der Subtraktion des ersten Winkels δ vom zweiten Winkel ε.

Als weitere Ausführungsform ist denkbar, dass das chirurgische Werkzeug ein Ultraschallschneidwerkzeug ist, bei welchem zwei definierte, verschiedene Winkelstellungen zwischen Effektor und Werkzeugschaft auf dieselbe Weise erzeugt werden, wie bei den vorangegangen beiden Ausführungsformen.

### Liste der Bezugszeichen

- 1: chirurgisches Werkzeug (erste Ausführungsform)
- 2: Werkzeugschaft
- 4: Effektoraufnahme
- 6: Effektor
- 8: Kopplungsstruktur
- 9: Handstück
- 10: Kopplungsabschnitt
- 11: Scharnier (am Effektor)
- 12: Arbeitsende
- 14: erste Kopplungsvorrichtung
- 16: zweite Kopplungsvorrichtung
- 17: chirurgisches Werkzeug (zweite Ausführungsform)
- 18: Werkzeugschaft
- 20: Effektoraufnahme
- 22: Effektor
- 24: Kopplungsabschnitt
- 26: Arbeitsende
- A: Längsachse des Werkzeugschaftes in der ersten Ausführungsform
- B: Längsachse der Effektoraufnahme in der ersten Ausführungsform
- C: Längsachse des Werkzeugschaftes in der zweiten Ausführungsform
- D: Längsachse des Werkzeugschaftes in der zweiten Ausführungsform

## Patentansprüche

1. Chirurgisches Werkzeug (1, 17), insbesondere chirurgische Transversalsäge, Stichsäge oder chirurgisches Ultraschallschneidwerkzeug, mit einem Effektor (6, 22) und einem Werkzeugschaft (2, 18), der an seinem proximalen Ende eine Kopplungsstruktur (8) zur Kopplung mit einem Handstück (9) aufweist und der zur reversiblen Kopplung des Werkzeugschafts (2, 18) mit dem Effektor (6, 22) an seinem distalen Ende eine Effektoraufnahme (4, 20) aufweist, welche zur Längsachse (A, C) des Werkzeugschafts (2, 18) in einem vorbestimmten ersten, insbesondere spitzen, Winkel (α, δ) angeordnet ist,
**dadurch gekennzeichnet, dass**
der Effektor (6, 22) über einen Kopplungsabschnitt (10, 24) durch eine Ausgestaltung der Effektoraufnahme derart, dass der Effektor durch eine Relativdrehung zwischen Werkzeugschaft (2, 18) und Effektor (6, 22) um die Werkzeugschaft-Längsachse (A, C) in genau zwei im 180° Drehabstand vorgesehenen Drehpositionen relativ zur Effektoraufnahme koppelbar ist, mit der Effektoraufnahme (4, 20) so gekoppelt werden kann, dass der Effektor (6, 22) und der Werkzeugschaft (2, 18) in zwei unterschiedliche Winkelstellungen (γ₁, γ₂, γ₃, γ₄) zueinander bringbar sind.

2. Chirurgisches Werkzeug (1, 17) nach Anspruch 1, **dadurch gekennzeichnet, dass**
der Effektor (6, 22) neben dem Kopplungsabschnitt (10, 24) ein Arbeitsende (12, 26) aufweist, welches mit dem Kopplungsabschnitt (10, 24) einen vorbestimmten zweiten Winkel (β, ε) einschließt.

3. Chirurgisches Werkzeug (1, 17) nach Anspruch 1, **dadurch gekennzeichnet, dass**
der Kopplungsabschnitt (10, 24) des Effektors (6, 22) mit der Effektoraufnahme (4, 20) eine Hinterschneidung ausbildet.

4. Chirurgisches Werkzeug (1, 17) nach Anspruch 1, **dadurch gekennzeichnet, dass**
die Effektoraufnahme (4, 20) eine erste Kopplungsvorrichtung (14) aufweist, die komplementär zu einer zweiten Kopplungsvorrichtung (16) am Kopplungsabschnitt (10, 24) des Effektors (6, 22) ausgebildet ist, sodass die erste und die zweite Kopplungsvorrichtung (14, 16) als Rastverbindung oder Schnappverbindung oder Steckverbindung zusammenwirken.

5. Chirurgisches Werkzeugsystem, das das chirurgisches Werkzeug (1, 17) nach Anspruch 1 und ein Handstück (9) zur Bedienung des chirurgischen Werkzeugs (1, 17) aufweist, **dadurch gekennzeichnet, dass**
der Effektor (6, 22) über einen Kopplungsabschnitt (10, 24) reversibel mit der Effektoraufnahme (4, 20) koppelbar ist und der Effektor (6, 22) und der Werkzeugschaft (2, 18) in zwei unterschiedliche Winkelstellungen (γ₁, γ₂, γ₃, γ₄) zueinander bringbar sind.

## Claims

1. Surgical tool (1, 17), in particular surgical transversal saw, jigsaw or surgical ultrasonic cutting tool, having an effector (6, 22) and a tool shaft (2, 18), which, has a coupling structure (8) at its proximal end for coupling to a handpiece (9) and which has an effector holder (4, 20) at its distal end for reversible coupling of the tool shaft (2, 18) with the effector (6, 22), the effector holder being arranged at a predetermined first, in particular acute, angle (α, δ) to the longitudinal axis (A, C) of the tool shaft (2, 18),
**characterized in that**
the effector (6, 22) can be coupled via a coupling section (10, 24) by configuring the effector holder such that the effector can be coupled relative to the effector holder by a relative rotation between tool shaft (2, 18) and effector (6, 22) about the longitudinal axis (A, C) of the tool shaft into exactly two rotational positions provided at a rotational distance by 180° in such a way that the effector (6, 22) and the tool shaft (2, 18) can be brought into at least two different angular positions (γ₁, γ₂, γ₃, γ₄ to each other.

2. The surgical tool (1, 17) according to claim 1, **characterized in that** the effector (6, 22) has, in addition to the coupling portion (10, 24), a working end (12, 26) which forms a predetermined second angle (β, ε) with the coupling portion (10, 24).

3. The surgical tool (1, 17) according to claim 1, **characterized in that**
the coupling portion (10, 24) of the effector (6, 22) forms an undercut with the effector holder (4, 20).

4. The surgical tool (1, 17) according to claim 1, **characterized in that**
the effector holder (4, 20) has a first coupling device (14) configured to complement a second coupling device (16) on the coupling portion (10, 24) of the effector (6, 22), so that the first and the second coupling device (14, 16) interact as a latching connection or snap connection or plug-in connection.

5. A surgical tool system comprising a surgical tool (1, 17) according to claim 1 and a handpiece (9) for operating the surgical tool (1, 17), **characterized in that**
the effector (6, 22) can be reversibly coupled to the effector holder (4, 20) via a coupling portion (10, 24) and the effector (6, 22) and the tool shaft (2, 18) can be brought into two different angular positions (γ₁, γ₂, γ₃, γ₄) relative to one another.

## Revendications

1. Outil chirurgical (1, 17), en particulier scie transversale, scie sauteuse chirurgicale ou outil de coupe à ultrasons chirurgical, avec un effecteur (6, 22) et une tige d'outil (2, 18) qui présente à son extrémité proximale une structure de couplage (8) pour le couplage avec une pièce à main (9) et qui présente, pour le couplage réversible de la tige d'outil (2, 18) avec l'effecteur (6, 22) à son extrémité distale, un logement d'effecteur (4, 20) qui est agencé selon un premier angle (α, δ) prédéterminé, en particulier aigu, par rapport à l'axe longitudinal (A, C) de la tige d'outil (2, 18),
**caractérisé en ce que**
l'effecteur (6, 22) peut être couplé par le biais d'une section de couplage (10, 24) par une configuration du logement d'effecteur de telle manière que l'effecteur puisse être couplé au logement d'effecteur (4, 20) par une rotation relative entre la tige d'outil (2, 18) et l'effecteur (6, 22) autour de l'axe longitudinal de tige d'outil (A, C) dans précisément deux positions de rotation prévues à une distance de rotation de 180° par rapport au logement d'effecteur, de sorte que l'effecteur (6, 22) et la tige d'outil (2, 18) puissent être amenés dans deux positions angulaires différentes (γ₁, γ₂, γ₃, γ₄) l'une par rapport à l'autre.

2. Outil chirurgical (1, 17) selon la revendication 1, **caractérisé en ce que**
l'effecteur (6, 22) présente à côté de la section de couplage (10, 24) une extrémité de travail (12, 26) qui forme avec la section de couplage (10, 24) un second angle prédéterminé (β, ε).

3. Outil chirurgical (1, 17) selon la revendication 1, **caractérisé en ce que**
la section de couplage (10, 24) de l'effecteur (6, 22) forme avec le logement d'effecteur (4, 20) une contre-dépouille.

4. Outil chirurgical (1, 17) selon la revendication 1, **caractérisé en ce que**
le logement d'effecteur (4, 20) présente un premier dispositif de couplage (14) qui est formé de manière complémentaire à un second dispositif de couplage (16) au niveau de la section de couplage (10, 24) de l'effecteur (6, 22) de sorte que le premier et le second dispositif de couplage (14, 16) interagissent comme liaison à encliquetage ou liaison à enclenchement ou connexion enfichable.

5. Système d'outil chirurgical qui présente l'outil chirurgical (1, 17) selon la revendication 1 et une pièce à main (9) pour la commande de l'outil chirurgical (1, 17), **caractérisé en ce que**
l'effecteur (6, 22) peut être couplé par le biais d'une section de couplage (10, 24) de manière réversible avec le logement d'effecteur (4, 20), et l'effecteur (6, 22) et la tige d'outil (2, 18) peuvent être amenés dans deux positions angulaires différentes (γ₁, γ₂, γ₃, γ₄) l'une par rapport à l'autre.
